(19) Europäisches Patentamt

European Patent Office

Office européen des brevets

(11) **EP 0 666 730 B1**

(12) **EUROPÄISCHE PATENTSCHRIFT**

(45) Veröffentlichungstag und Bekanntmachung des Hinweises auf die Patenterteilung:
**20.05.1998 Patentblatt 1998/21**

(21) Anmeldenummer: 93923565.1

(22) Anmeldetag: **28.10.1993**

(51) Int. Cl.$^6$: **A61K 7/16**, A61K 7/18

(86) Internationale Anmeldenummer:
**PCT/EP93/02991**

(87) Internationale Veröffentlichungsnummer:
**WO 94/10969 (26.05.1994 Gazette 1994/12)**

(54) **REMINERALISIERENDES ZAHNPFLEGEMITTEL**

REMINERALIZING TOOTH-CARE AGENT

DENTIFRICE REMINERALISANT

(84) Benannte Vertragsstaaten:
**AT BE CH DE DK ES FR GB GR IT LI NL PT SE**

(30) Priorität: **06.11.1992 DE 4237500**

(43) Veröffentlichungstag der Anmeldung:
**16.08.1995 Patentblatt 1995/33**

(73) Patentinhaber:
**Henkel Kommanditgesellschaft auf Aktien
40191 Düsseldorf (DE)**

(72) Erfinder:
- **SCHUHMANN, Klaus**
  **D-40699 Erkrath (DE)**
- **FÖRG, Franz**
  **D-40764 Langenfeld (DE)**
- **LASKA, Hans**
  **D-40627 Düsseldorf (DE)**

(56) Entgegenhaltungen:
EP-A- 0 040 938          DE-A- 2 526 808
FR-A- 2 495 467          FR-A- 2 556 962
GB-A- 2 124 902          US-A- 4 024 239

**Beschreibung**

Die Erfindung betrifft Zahnpflegemittel mit einer die Zahnoberfläche restaurierenden Wirkung sowie ein Verfahren zur Restaurierung der Zahnoberfläche.

Zahnpflegemittel dienen in erster Linie der Reinigung der Zahnoberflächen von Speiseresten, Verfärbungen und fest anhaftenden bakteriellen Zahnbelägen. Darüber hinaus wird versucht, durch spezielle Zusätze, z.B. durch Fluorverbindungen oder antimikrobielle Stoffe Zahnerkrankungen wie Karies oder Parodontose vorzubeugen.

Als eines der ersten Stadien der Zahnkaries werden Läsionen im Zahnschmelz und offene Dentinkanälchen (sog. Tomes Pits) beobachtet, die durch Lösungsvorgänge unter dem Einfluß von säurebildenden Bakterien entstehen. Diese Störungen der Dentinsubstanz machen sich z.B. durch Zahnhalssensibilität gegenüber Temperaturschwankungen bemerkbar. Während durch Zusätze von desensibilisierenden Wirkstoffen lediglich die schmerzhaften Symptome bekämpft werden, hat man durch Zusätze, welche die Apatitlöslichkeit reduzieren, bereits versucht, die Bildung solcher Zahnoberflächen-Läsionen zu verhindern.

Aus US-A-4,024,239 sind Zahnpasten bekannt, die Dicalciumphosphat-dihydrat zur Stabilisierung und zum Schutz der Aluminiumtube enthalten. Auch in GB-A-2 124 902 wird ein Zusatz von Dicalciumphosphat für diesen Zweck empfohlen. DE-A-2 526 808 beschreibt Zahnpasten für Druckgasverpackung, die als Fluorkomponente Magnesiummonofluorphosphat enthalten. Aus FR-A-2 556 962 waren schließlich Zahnpasten bekannt, die in Verbindung mit einem Bindemittelsystem auf Basis von I-Carraghenan wenigstens 20 Gew.-% an Dicalciumphosphat als Polierkomponente zur Verbesserung der rheologischen Eigenschaften enthalten.

In neuerer Zeit wurden auch bereits Vorschläge gemacht, vorhandene Schäden durch remineralisierende Zahnpflegemittel zu verringern. So wurde von Chow und Brown (in J. Dent. Res., 54, (1975), 65 - 70) vorgeschlagen, Dicalciumphosphat-dihydrat zur Remineralisation des Dentins einzusetzen. Aus US 4,097,588 war ein Mundwasser mit remineralisierender Wirkung bekannt, das mit $CaHPO_4 \cdot 2H_2O$ gesättigt war.

Die bisher bekannten Versuche, die Zahnoberfläche auf diese Weise zu restaurieren, führen aber zu einem unkontrollierten Wachstum von Hydroxylapatit-Kristallen auf der Zahnoberfläche, das einem weiteren Angriff auf die Zahnoberfläche wenig Widerstand bietet. Es war daher ein Ziel der vorliegenden Erfindung, eine Zahnpaste zu entwickeln, die eine kontrollierte Remineralisation, insbesondere in den Kratzrillen und Dentinkanälen, bewirkt und diese weitgehend einebnet, so daß eine geschlossene, glatte Zahnoberfläche gebildet wird.

Es wurde gefunden, daß dieses Ziel durch die erfindungsgemäße Poliermittelkombination in hohem Maße erreicht wird.

Gegenstand der Erfindung ist eine Zahnpaste zur Restaurierung der Zahnoberfläche mit einem Gehalt an Poliermitteln, Fluorverbindungen, Feuchthaltemitteln, Bindemitteln und Wasser, die als Poliermittel eine Kombination von Kieselsäure und Dicalciumphosphat-dihydrat (Brushit) im Gewichtsverhältnis von 10 : 1 bis 1 : 1 enthält. Es wurde beobachtet, daß durch regelmäßige Behandlung der Zähne mit der erfindungsgemäßen Zahnpaste ein Verschluß der Dentinkanäle unter weitgehender Einebnung der Zahnoberfläche erreicht wird.

Als Kieselsäurepoliermittel eignen sich alle als Putzkörper bekannten Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten, wie sie z.B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 $m^2$/g, einer Partikelgröße von 0,5 - 20 μm (μ) wobei wenigstens 80 Gew.-% der Primärpartikel unter 5 μm (μ) liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste.

Bevorzugt geeignete Fällungskieselsäuren weisen außerdem gerundete Ecken und Kanten auf und sind unter der Handelsbezeichnung Sident[R] 12 DS (DEGUSSA) erhältlich.

Um einen genügend hohen Anteil an Kieselsäuren mit einer mittleren Teilchengröße von weniger als 5 μ und besonders um einen Anteil von wenigstens 3 Gew.-%, bezogen auf die gesamte Zahnpaste, an Kieselsäure mit einer Primärpartikelgröße von 1 - 3 μm (μ) zu erhalten, setzt man bevorzugt neben der genannten Fällungskieselsäure noch eine feinteiligere Type mit einer BET-Oberfläche von 150 - 250 ein. Eine geeignete Kieselsauretype ist z.B. Sipernat[R] 22LS (DEGUSSA), die in einer Menge von 1 - 5 Gew.-% der Zahnpaste eingesetzt wird.

Es hat sich als besonders vorteilhaft für die Ausbildung einer glatten Zahnoberfläche erwiesen, wenn die erfindungsgemäße Zahnpaste geringe Mengen von frisch gefällter, d.h. in situ bei der Pastenherstellung erzeugter Kieselsäure enthält. Dies wird z.B. dadurch erreicht, daß man bei der Herstellung der Zahnpaste durch z.B. Citronensäure einen pH-Wert von 3 - 5 einhält und dann durch Zusatz geringer Mengen einer wäßrigen Natriumsilikatlösung den pH-Wert auf 7 - 7,5 anhebt. Die auf diese Weise in situ gebildete Kieselsäure ist äußerst feinteilig und macht weniger als 0,1 Gew.-% der Zahnpaste aus.

Neben Kieselsäurepoliermitteln können in geringerem Umfang auch andere bekannte Polierstoffe enthalten sein, z.B. Aluminiumoxid in Form von schwach calcinierter Tonerde mit einem Gehalt an $\gamma$- und $\alpha$-Aluminiumoxid. Ein solches Aluminiumoxid ist unter der Handelsbezeichnung Poliertonerde P10 feinst (Giulini-Chemie) erhältlich. Das Gewichtsverhältnis solcher gegebenenfalls zusätzlichen Poliermittelkomponenten zum Kieselsäure-Poliermittel sollte jedoch nicht höher als (1 - 15) : 100 sein.

Die zweite, obligatorische Poliermittelkomponente ist Dicalciumphosphat-dihydrat ($CaHPO_4 \cdot 2H_2O$), das in der Natur als Brushit vorkommt. Dicalciumphosphat-dihydrat wird seit langem als Poliermittel in Zahnpasten verwendet und ist ebenfalls in geeigneten Korngrößen von 1 - 50 $\mu$ im Handel erhältlich. Die bevorzugte Einsatzmenge beträgt 1 - 5 Gew.-% der Zahnpaste.

Die erfindungsgemäßen Zahnpasten sollten möglichst keine weiteren Calcium-Ionen-bildenden Polierkomponenten wie z.B. Kreide oder andere Calciumphosphate enthalten.

Es wurde hingegen beobachtet, daß Magnesiumionen einen günstigen Einfluß auf das kontrollierte Kristallwachstum des in den Zahnläsionen wachsenden Hydroxylapatit haben, die beschleunigte Härtung des Zahnemails fördern, und daher einen weiteren Beitrag zur Lösung der erfindungsgemäßen Aufgabe leisten. Ein weiterer Gegenstand der Erfindung ist daher eine erfindungsgemäße Zahnpaste, die ein wasserlösliches Magnesiumsalz in einer Menge entsprechend 0,1 - 0,5 Gew.-% Mg enthält. Als Magnesiumsalze eignen sich alle Salze, die in Wasser bei 20°C wenigstens zu 0,5 Gew.-% (berechnet als Mg) löslich sind, z.B. $MgSO_4$, $MgCl_2$ und Magnesium-monofluorophosphat ($MgPO_3F$).

Es wurde weiterhin festgestellt, daß Fluorophosphat-Ionen einen günstigen Einfluß auf das kontrollierte Kristallwachstum des Hydroxylapatits haben. Bevorzugt sind daher erfindungsgemäße Zahnpasten, die als Fluorverbindung ein Monofluorophosphat in einer Menge entsprechend 0,2 - 2,0 Gew.-% ($PO_3F^-$) enthalten. Es eignen sich hierfür z.B. die Alkalisalze, z.B. das handelsübliche Natrium-monofluorophosphat. Der bevorzugte Gehalt an Magnesiumionen und an Fluorophosphationen läßt sich besonders vorteilhaft dadurch erreichen, daß als Fluorverbindung Magnesiummonofluorophosphat enthalten ist.

Als Träger für die erfindungsgemäßen Zahnpasten, der die Einstellung einer geeigneten Konsistenz für die Dosierung aus Tuben, Spendebehältern oder flexiblen Flaschen auf der Grundlage der erfindungsgemäßen Poliermittelkombination ermöglicht, eignet sich eine Kombination aus Feuchthaltemitteln, Bindemitteln und Wasser. Als Feuchthaltemittel können z.B. Glycerin, Sorbit, Xylit, Propylenglycole, Polyethylenglycole, insbesondere solche mit mittleren Molekulargewichten von 200 - 800 eingesetzt werden. Als Konsistenzregler (bzw. Bindemittel) dienen z.B. natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthangum, Johannisbrotmehl, Pectine, wasserlösliche Carboxyvinylpolymere (z.B. Carbopol$^{(R)}$-Typen), Polyvinylalkohol, Polyvinylpyrrolidon, Polyethylenglycole, insbesondere solche mit Molekulargewichten von 1 500 - 1 000 000.

Weitere Stoffe, die sich zur Viskositätskontrolle eignen, sind z.B. Schichtsilikate wie z.B. Montmorillonit-Tone, kolloidale Verdickungskieselsäuren wie z.B. Aerogel-Kieselsäuren, pyrogene Kieselsäuren oder feinstvermahlene Fällungskieselsäuren. Ein für die Herstellung von Zahnpasten mit der erfindungsgemäßen Poliermittelkombination besonders gut geeigneter Träger enthält

15 - 25 Gew.-% Glycerin
10 - 20 Gew.-% Sorbit
1 - 5 Gew.-% Polyethylenglycol (MG : 400)
1 - 5 Gew.-% Verdickungskieselsäure
0,1 - 1 Gew.-% Xanthan-Gum und
35 - 45 Gew.-% Wasser.

Die Zahnpasten können in ihrer Viskosität auch so niedrig eingestellt sein, daß sie sich als "flüssige Zahnreinigungsmittel" mit einer Viskosität von 2 000 - 10 000 m · Pa · s (25°C) aus einer flexiblen Kunststofflasche auf die Zahnbürste dosieren lassen, dort zwischen die Borsten eindringen, aber nicht von der Zahnbürste abtropfen. Für diesen Zweck eignet sich als Bindemittel bevorzugt eine Kombination aus 0,1 - 1 Gew.-% Xanthan-Gum und 0,01 - 5 Gew.-% eines viskositätsstabilisierenden Zusatzes aus der Gruppe

- der hydroxypropylsubstituierten Hydrocolloide oder
- der Polyethylenglycol/Polypropylenglycol Copolymere

mit einem mittleren Molekulargewicht von 1 000 bis 5 000.

Weitere übliche Zahnpastenzusätze, die insgesamt bis zu ca. 5 Gew.-% der Zahnpastenzusammensetzung ausmachen können, sind z.B. Tenside zur Unterstützung der Reinigungswirkung und ggf. zur Entwicklung von Schaum beim Zähnebürsten und zur Stabilisierung der Dispersion der Poliermittelkomponenten in dem Träger. Geeignete Tenside sind z.B. lineare Natriumalkylsulfate mit 12 - 18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von linearem Alkan($C_{12}$-$C_{18}$)-sulfonat, von Sulfobernsteinsäuremonoalkyl($C_{12}$-$C_{18}$)-estern, von sulfatierten Fettsäuremonoglyceriden, sulfatierten Fettsäurealkanolamiden, Sulfoessigsäurealkyl($C_{12}$-$C_{16}$)-estern, Acylsarcosinen, Acyltauriden und Acylisethionaten mit jeweils 8 - 18 C-Atomen in der Acylgruppe. Auch zwitterionische, ampholytische und nichtionische Tenside sind geeignet, z.B. Oxethylate von Fettsäuremono- und -diglyceriden, von Fettsäure-Sorbitanestern und Alkyl(oligo)-Glucoside.

Weitere übliche Zahnpastenzusätze sind

- Konservierungsmittel und antimikrobielle Stoffe, wie z.B. p-Hydroxybenzoesäuremethyl-, -ethyl- oder propylester, Natriumsorbat, Natriumbenzoat, Bromchlorophen, Phenyl-salicylsäureester, Biguanide, Thymol usw.

- Süßungsmittel wie z.B. Saccharin-Natrium, Natrium-Cyclamat, Sucrose, Lactose, Maltose, Fructose,

- Aromen wie z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen,

- Pigmente wie z.B. Titandioxid

- Farbstoffe

- Puffersubstanzen wie z.B. primäre, sekundäre oder tertiäre Alkaliphosphate oder Citronensäure/Natriumcitrat,

- wundheilende und entzündungshemmende Stoffe wie z.B. Allantoin, Harnstoff sowie Azulen, Kamillewirkstoffe, Acetylsalicylsäurederivate.

Die folgenden Beispiele sollen den Erfindungsgegenstand näher erläutern:

**Beispiele**

Es wurden die erfindungsgemäßen Zahnpasten (Beispiele 1, 2 und 3) und eine Vergleichszahnpaste (Beispiel V) hergestellt (vgl. Tabelle I).

Tabelle I

| Bestandteile | 1 | 2 | 3 | V |
|---|---|---|---|---|
| Fällungskieselsäure: Sident® 12 DS | 12,0 | 12,0 | 12,0 | 12,0 |
| Fällungskieselsäure: Sipernat® 22 LS | 3,0 | 3,0 | 3,0 | 6,0 |
| Dicalciumphosphat-dihydrat | 4,0 | 4,0 | 4,0 | - |
| $MgSO_4 \cdot 7H_2O$ | 1,4 | 1,4 | - | - |
| Natrium-monofluorophosphat $NaPO_3F$ | 0,8 | 0,8 | 0,8 | 0,8 |
| Glycerin 86 %, DAB (ber. wasserfrei) | 18,0 | 18,0 | 18,0 | 18,0 |
| Sorbit 70 %, DAB (ber. wasserfrei) | 14,0 | 14,0 | 14,0 | 14,0 |
| Polyethylenglycol (MG : 400) | 2,0 | 2,0 | 2,0 | 2,0 |
| Verdickungskieselsäure (FK 320 DS) | 1,0 | 1,33 | 1,33 | 1,33 |
| Xanthan-Gum (Keltrol® F) | 0,54 | 0,54 | 0,54 | 0,71 |
| Titandioxid (Typ Anatas) | 1,0 | 1,0 | 1,0 | 1,0 |

Tabelle I (fortgesetzt)

| Bestandteile | 1 | 2 | 3 | V |
|---|---|---|---|---|
| Natrium-Laurylsulfat (Texapon® K 1296) | 1,44 | 1,44 | 1,44 | 1,44 |
| Natrium-Wasserglas (ber. als $Na_2SiO_3$) | 0,06 | - | - | - |
| Natrium-hydroxid | - | 0,01 | 0,01 | 0,01 |
| Citronensäure( $\cdot$ 1 $H_2O$) | 0,03 | 0,03 | 0,03 | 0,03 |
| Saccharin | 0,2 | 0,2 | 0,2 | - |
| Geschmackstoff | 0,8 | 0,8 | 0,8 | 0,8 |
| Wasser | 39,73 | 39,45 | 40,85 | 41,88 |

Es wurden folgende Handelsprodukte verwendet:

Sident® 12 DS: Fällungskieselsäure der Firma DEGUSSA AG Frankfurt a.M.,

| BET-Oberflä-che: | 80 $m^2$/g |
|---|---|
| Stampfdichte: | 220 g/l |

Sipernat® 22 LS: Fällungskieselsäure der Firma DEGUSSA AG Frankfurt a.M.,

| BET-Oberflä-che: | 190 $m^2$/g |
|---|---|
| Stampfdichte: | 80 g/l |

Kieselsäure FK 320 DS: Fällungskieselsäure der Firma DEGUSSA AG Frankfurt a.M.,

| BET-Oberfläche: | 170 m2/g |
|---|---|
| Stampfdichte: | 80 g/l |

Keltrol® F: Xanthan-Gum der Firma KELCO, Brüssel

Texapon K 1296-Granulat: Na-Laurylsulfat von HENKEL KGaA Düsseldorf

**Wirkungsnachweis**

Mit der erfindungsgemäßen Zahnpaste (Beispiel 1) und der Vergleichszahnpaste (Beispiel V, ohne Dicalciumphosphat-dihydrat und ohne Magnesiumsulfat) wurden die folgenden Versuche durchgeführt:

Aus einem Humanzahn wurden 4 x 5 mm große Stücke (sog. Slabs) aus der Krone (für Versuche mit Schmelz) und aus dem Wurzelbereich (für Versuche mit Dentin) herausgesägt und die Oberfläche mit Naßschleifpapier geebnet und mit Polierpaste glatt poliert.

Die Schmelz-Slabs wurden zusätzlich mit 0,1 molarer wäßriger Milchsäure, die 500 mg/l Hydroxylapatit enthielt, bei pH = 4,6 und T = 37°C über 6 Stunden geätzt.

Die so vorbereiteten Proben wurden dann täglich zweimal 5 Minuten mit der Test-Zahnpaste (verdünnt mit Wasser 1 : 1) geputzt. In der übrigen Zeit wurden die Proben bei 37°C in einer $CaHPO_4 \cdot 2H_2O$-Suspension gelagert, die zwei-

mal täglich (morgens und abends) durch Fällung

$$(CaCl_2 + Na_2HPO_4 \rightarrow CaHPO_4 + 2NaCl)$$

frisch hergestellt wurde. Diese Zahnbehandlung wurde jeweils 20 Tage lang durchgeführt.

Vor der Behandlung (d.h. nach dem Polieren der Slabs) und nach Ende der Behandlung wurden rasterelektronenmikroskopische (REM) Aufnahmen der Probenoberfläche gemacht.

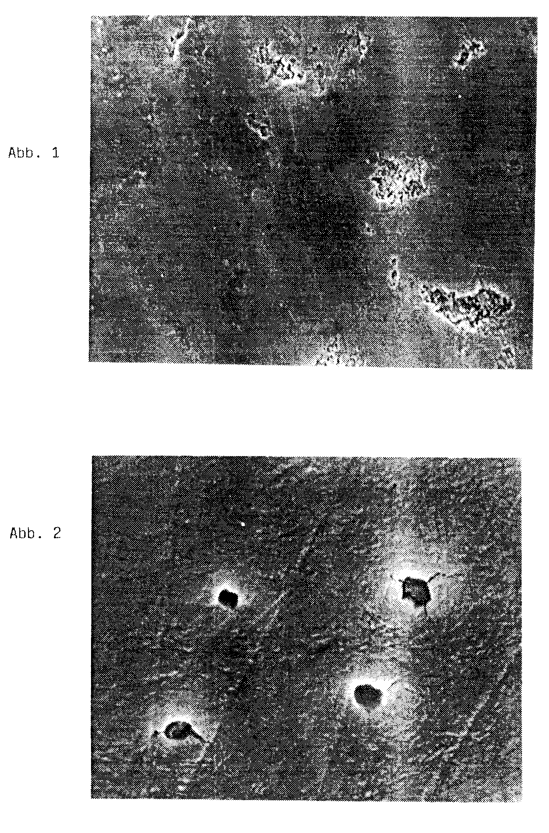

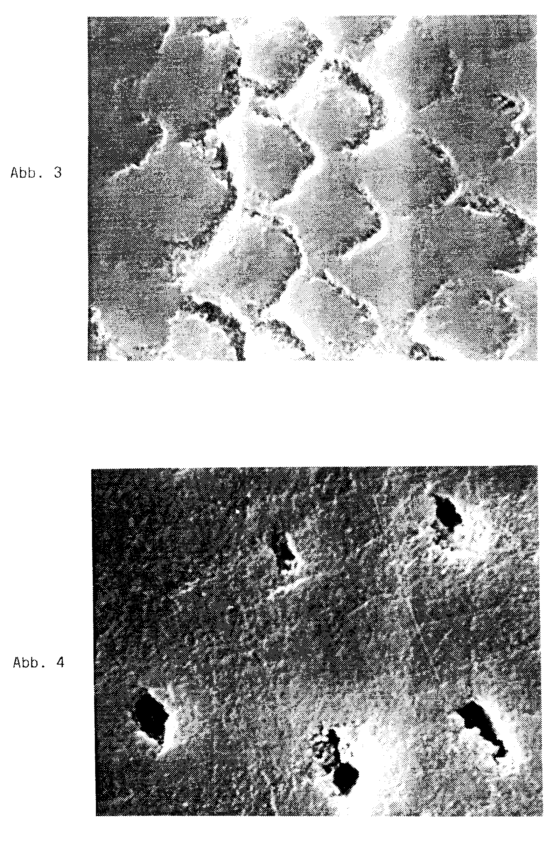

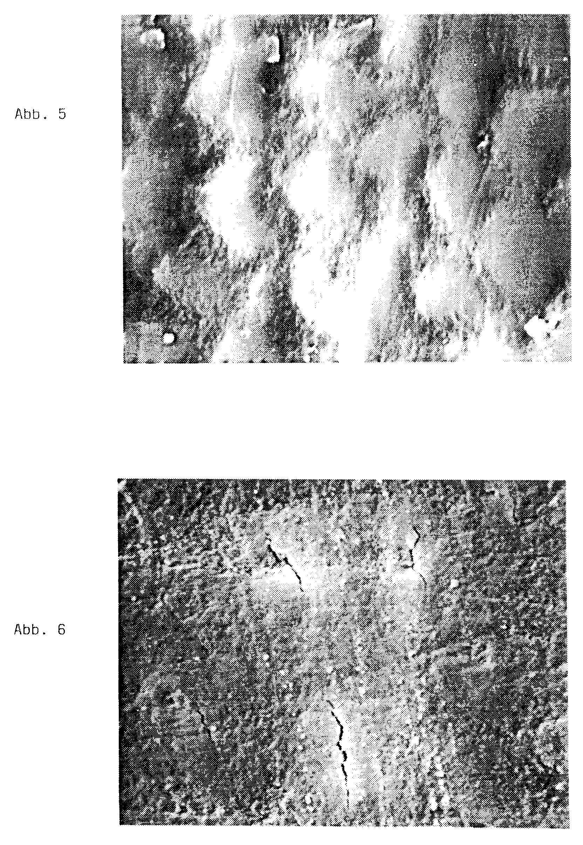

Auf den Abbildungen 1 - 6 sind diese im Maßstab 5000 : 1 wiedergegeben.

Abb. 1:     Humanschmelz, unbehandelt
Abb. 2:     Humandentin, unbehandelt
Abb. 3:     Humanschmelz, behandelt mit Vergleichszahnpaste V
Abb. 4:     Humandentin, behandelt mit Vergleichspaste V
Abb. 5:     Humanschmelz, behandelt mit Zahnpaste Beispiel 1. Man erkennt, daß sich in den Rillen der Oberflächenstruktur Hydroxylapatit abgeschieden hat.
Abb. 6:     Humandentin, behandelt mit Zahnpaste Beispiel 1. Man erkennt deutlich den weitgehenden Verschluß der Dentinkanäle durch abgeschiedenen Hydroxylapatit.

**Patentansprüche**

1.  Zahnpaste zur Restaurierung der Zahnoberfläche mit einem Gehalt an Poliermitteln, Fluorverbindungen, Feuchthaltemitteln, Bindemitteln und Wasser, dadurch gekennzeichnet, daß als Poliermittel eine Kombination von Kieselsäuren und Dicalciumphosphat-dihydrat (Brushit) im Gewichtsverhältnis von 10 : 1 bis 1 : 1 enthalten ist.

2.  Zahnpaste nach Anspruch 1, dadurch gekennzeichnet, daß ein wasserlösliches Magnesiumsalz in einer Menge entsprechend 0,1 - 0,5 Gew.-% Magnesium enthalten ist.

3.  Zahnpaste nach Anspruch 1 oder 2, dadurch gekennzeichnet, daß als Fluorverbindung ein Monofluorophosphat in einer Menge entsprechend 0,2 - 2,0 Gew.-% ($PO_3F^-$) enthalten ist.

4.  Zahnpaste nach Anspruch 2 oder 3, dadurch gekennzeichnet, daß als Fluorverbindung Magnesium-monofluorophosphat enthalten ist.

5.  Zahnpaste nach Anspruch 1 bis 4, dadurch gekennzeichnet, daß als Poliermittel eine Fällungskieselsäure mit einer mittleren Teilchengröße von 0,5 - 30 $\mu$m ($\mu$) in einer Menge von 10 - 20 Gew.-% und Dicalciumphosphat-dihydrat (Brushit) in einer Menge von 1 - 5 Gew.-% enthalten ist.

**Claims**

1.  A toothpaste for restoring the surface of teeth containing polishes, fluorine compounds, humectants, binders and water, characterized in that it contains a combination of silicas and dicalcium phosphate dihydrate (brushite) in a ratio by weight of 10:1 to 1:1 as the polishing component.

2.  A toothpaste as claimed in claim 1, characterized in that it contains a water-soluble magnesium salt in a quantity corresponding to 0.1 to 0.5% by weight of magnesium.

3.  A toothpaste as claimed in claim 1 or 2, characterized in that it contains a monofluorophosphate in a quantity corresponding to 0.2 to 2.0% by weight of ($PO_3F^-$) as the fluorine compound.

4.  A toothpaste as claimed in claim 2 or 3, characterized in that it contains magnesium monofluorophosphate as the fluorine compound.

5.  A toothpaste as claimed in claims 1 to 4, characterized in that it contains a precipitated silica with an average particle size of 0.5 to 30 $\mu$m ($\mu$) in a quantity of 10 to 20% by weight and dicalcium phosphate dihydrate (brushite) in a quantity of 1 to 5% by weight as polishes.

**Revendications**

1. Dentifrice pour la restauration de la surface des dents contenant des agents de polissage, des composés fluorés, des agents humidifiants, des liants, de l'eau
caractérisé en ce qu'
il contient comme agents de polissage une combinaison d'acides siliciques et de phosphate dicalcique dihydraté (brushite) dans un rapport pondéral de 10:1 à 1:1.

2. Dentifrice selon la revendication 1,
caractérisé en ce qu'
il contient un sel de magnésium soluble dans l'eau en une quantité correspondant à 0,1-0,5 % en poids de magnésium.

3. Dentifrice selon la revendication 1 ou 2,
caractérisé en ce qu'
il contient comme composé fluoré un monofluorophosphate en une quantité correspondant à 0,2-2 % en poids $(PO_3F^-)$.

4. Dentifrice selon la revendication 2 ou 3,
caractérisé en ce qu'
il contient comme composé fluoré le monofluorophosphate de magnésium.

5. Dentifrice selon la revendication 1 à 4,
caractérisé en ce qu'
il contient comme agent de polissage un acide silicique de précipitation ayant une taille particulaire moyenne de 0,5-30 $\mu$m en une quantité de 10-20 % en poids et du phosphate dicalcique dihydraté (brushite) en une quantité de 1-5 % en poids.

Abb. 1

Abb. 2

Abb. 3

Abb. 4

Abb. 5

Abb. 6